# EUROPEAN PATENT APPLICATION

(11) **EP 0 889 055 A1**
(43) Date of publication of application: **07.01.1999**
(21) Application number: 98900165.6
(22) Date of filing: 07.01.1998
(51) Int. Cl.: C08B 37/08

(54) **OLIGOSULFATED HYALURONIC ACID**

(30) Priority: 10.01.1997 JP 13223/97
(71) Applicant: SHISEIDO COMPANY LIMITED, Tokyo 104-10 (JP)
(72) Inventor: AKIMA, Kazuo, Shiseido Research Center, Yokohama-shi, Kanagawa 223-8553 (JP); YOSHIDA, Yuzo, Shiseido Research Center, Yokohama-shi, Kanagawa 223-8553 (JP); KITAMURA, Kenji, Shiseido Research Center, Yokohama-shi, Kanagawa 223-8553 (JP); MUGIKURA, Shigeru, Shiseido Research Center, Yokohama-shi, Kanagawa 223-8553 (JP); SHIRATORI, Kohya, Shiseido Research Center, Yokohama-shi, Kanagawa 223-8553 (JP); TANAKA, Chiaki, Shiseido Research Center, Yokohama-shi, Kanagawa 223-8553 (JP); HORII, Izumi, Shiseido Research Center, Yokohama-shi, Kanagawa 223-8553 (JP)
(74) Representative: Kraus, Walter, Dr.
(86) International application number: JP9800018
(87) International publication number: WO9830595

(57) **Abstract**

Disclosed is a sulfated oligohyaluronate having an average molecular weight of 1,000 to 50,000 and containing 10 to 90% of sulfated hydroxyl groups. This sulfated oligohyaluronate has excellent skin permeability and can hence be used as an active ingredient in the technical fields of cosmetics and dermatology.

## Description

### Technical Field

This invention relates to sulfated oligohyaluronates. The sulfated oligohyaluronates of the present invention are particularly useful as active ingredients in the technical fields of cosmetics and dermatology.

### Background Art

Hyaluronic acid is a straight-chain high-molecular polysaccharide composed of β-D-N-acetylglucosamine and β-D-glucuronic acid which are alternately joined by covalent bonds. Because of its rather easy availability among mucopolysaccharides and its unique physicochemical and physiological properties, hyaluronic acid itself and its various derivatives are being used for therapeutic and cosmetic purposes. Moreover, a variety of new uses thereof have been proposed. For example, the present inventors have found that hyaluronic acid sulfate which is a derivative of hyaluronic acid exhibits a powerful anti-inflammatory effect on an inflammation mediated by selectin serving as an adhesion factor, and have proposed a new type of anti-inflammatory agent (Japanese Patent Application No. 40309/'96 and International Publication of PCT Application No. WO96/24362).

Some of the above-described applications of hyaluronic acid itself and its derivatives utilize their properties resulting from macromolecules having a molecular weight of several hundreds of thousands or greater than one million. However, in applications (e.g., external preparations) which require some degree of permeability through certain tissues (e.g., the skin) of living organisms, hyaluronic acid and its derivatives which consist of macromolecules as described above may not always be used advantageously.

On the other hand, it has been suggested that, among mucopolysaccharide sulfates having a relatively low molecular weight, the compounds formed by further sulfation of chondoitins and having a molecular weight of 17,000 are promising as ingredients for cosmetics because of their excellent percutaneous absorbability, good moisture-retaining effect and the like (see, for example, "Shukan Shogyo" published on May 22, 1995). Chondroitins are composed of disaccharide repeating units comprising N-acetyl-D-galactosamine and D-glucuronic acid, and they are epimers of hyaluronic acid in which the hydroxyl group at the 4-position of β-D-N-acetylglucosamine constituting the repeating units has a different steric configuration.

As compared with chondroitins, hyaluronic acid sulfate is readily available and, moreover, has very interesting physiological activities as described above. Thus, it would be desirable to provide such a compound having excellent permeability through the tissues of living organisms.

Accordingly, an object of the present invention is to provide a hyaluronic acid derivative having improved permeability through the tissues of living organisms (in particular, the skin) while substantially retaining the physiological activities and physicochemical properties possessed by high-molecular weight hyaluronic acid sulfate.

### Disclosure of the Invention

As a result of investigations on the skin permeability of hyaluronic acid sulfate which varies with its molecular weight, the present inventors have now found that, in contrast to the properties of the sulfated products of chondroitins having a molecular weight of 17,000 as suggested in the aforementioned "Shukan Shogyo" published on May 22, 1995, hyaluronic acid sulfate in the form of an oligomer having a viscosity-average molecular weight of not greater than about 10,000 has excellent skin permeability similarly to unsulfated hyaluronic acid, and it also exhibits moisture-retaining properties and certain physiological activities. Moreover, it has also be found that, where a moisture-retaining effect, moistening of the skin surface, and the like are chiefly aimed at, even a sulfated oligohyaluronate having a viscosity-average molecular weight of greater than 10,000 and up to about 50,000 may be suitably used in external preparations for application to the skin. Furthermore, such sulfated oligohyaluronates are not described in the existing technical literature.

Accordingly, the present invention provides a sulfated oligohyaluronate composed of repeating units represented by the following formula (I) or a salt thereof. 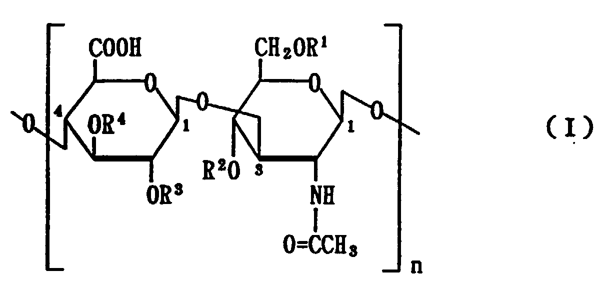 wherein
R¹, R², R³ and R⁴ each independently represent a hydrogen atom or a SO₃H group:
n represents an average value corresponding to a viscosity-average molecular weight of 1,000 to 50,000; and
10 to 90% of the total number of the radicals represented by R¹, R², R³ and R⁴ are occupied by SO₃H groups.

### Brief Description of the Drawing

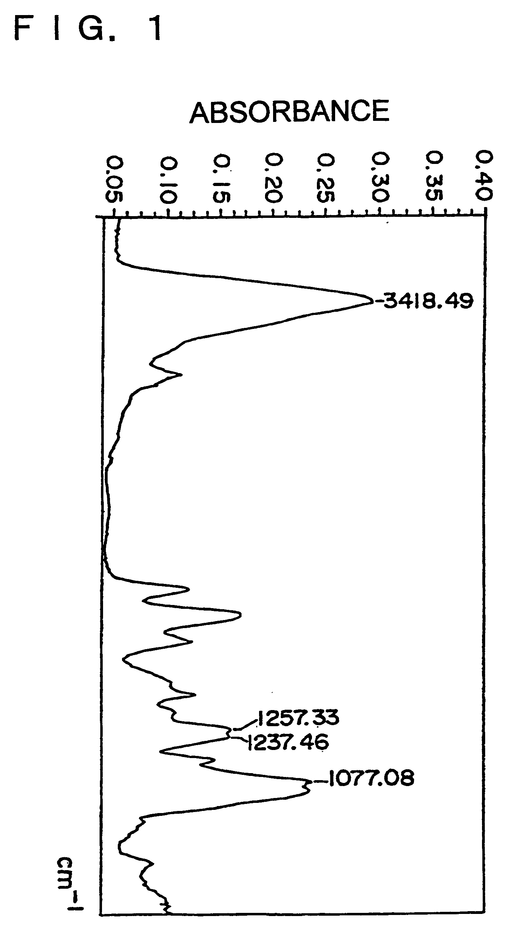

FIG. 1 shows the infrared absorption spectrum of a typical sulfated oligohyaluronate in accordance with the present invention (which was obtained in Example 2).

### Detailed Description of the Invention

According to the present invention, the average value of n in the above formula (I) corresponds not only to the peak value of an oligomer mixture showing substantial monodispersity, but also to the peak value of an oligomer mixture showing polydispersity. That is, the sulfated oligohyaluronates of the present invention include any oligomer mixture in which, when the distribution of the molecular weights of oligomers is estimated, for example, from the intrinsic viscosity, the peak value of the distribution falls within the range of 1,000 to 50,000.

Moreover, as to the proportion (or the degree of substitution) of SO₃H groups represented by R¹, R², R³ and R⁴ in formula (I), the sulfated oligohyaluronates of the present invention include any oligomer mixture in which, on the basis of all oligomers (and not the repeating units of each oligomer), 10 to 90% of the hydrogen atoms of all hydroxyl groups present in the hyaluronic acid oligomers are replaced by SO₃H groups. Accordingly, the SO₃H groups may be unevenly distributed in the oligomers. However, it is usually preferable from the viewpoint of preparation and use that the SO₃H groups are evenly distributed through the molecule.

At each end of the oligohyaluronic acid sulfate molecule composed of repeating units represented by formula (I), a hydrogen atom, an SO₃H group, or a sugar residue derived from either β-D-N-acetylglucosamine or β-D-glucuronic acid in formula (I) is covalently bonded to the indicated oxygen atom. Among others, sulfated oligohyaluronates having a hydrogen atom or an SO₃H group covalently bonded to either end of the molecule are preferred. The reason for this is that, as will be described later, oligohyaluronic acids used as starting materials for the sulfated oligohyaluronates of the present material may conveniently be prepared by treating high-molecular weight hyaluronic acid with hyaluronidase.

Where a sulfated oligohyaluronate in accordance with the present material is applied to the skin in the field of cosmetics and the like, the aforesaid numeral n should preferably have a value corresponding to a viscosity-average molecular weight of 1,000 to 10,000 and more preferably 2,000 to 7,000, though the present invention is not limited thereto. The reason for this is that such a sulfated oligohyaluronate exhibits suitable skin permeability and, moreover, has a certain moisture-retaining effect. Where skin permeability is chiefly desired, the sulfated oligohyaluronate should preferably have a value of n corresponding to a viscosity-average molecular weight of 5,000 or less.

The number of repeating units (n) in formula (I) may vary with the degree of substitution of SO₃H groups, i.e., the degree of sulfation. However, n typically has a value of about 6 to 10. For the purpose of application to the skin, a sulfated oligohyaluronate composed of such a number of repeating units and having a degree of sulfation of 20 to 50% is especially preferred.

The sulfated oligohyaluronates of the present invention may also exist in the form of salts with an alkali metal or alkaline earth, preferably sodium, potassium or lithium, or salts with ammonia or an organic amine.

In order to obtain the above-described sulfated oligohyaluronates of the present invention or their salts, the sulfated oligohyaluronates may be prepared from the corresponding oligohyaluronic acids according to a per se known sulfation process. Furthermore, if desired, the corresponding salts may be prepared by subjecting the resulting sulfated products to a salt forming process using a hydroxide or carbonate of an alkali metal, or ammonia or an organic amine.

An oligohyaluronic acid may be prepared by subjecting high-molecular weight hyaluronic acid (for example, with a molecular weight of 2,000 kDa) obtained from any of various natural sources to a treatment under high shear stresses or a treatment with an enzyme (hyaluronidase) (see, for example, A. Sattor et al., J. Invest. Dermatol., Vol. 103: 576-579, 1994). The enzyme treatment is favorable in that an oligohyaluronic acid having a narrow molecular weight distribution can be obtained by properly choosing the enzyme activity used and the reaction time used. Moreover, in the average molecular weight range in accordance with the present invention, a substantially monodisperse oligohyaluronic acid can be obtained by resorting to a treatment such as gel filtration or fractional precipitation using an organic solvent such as acetone.

The oligohyaluronic acid thus obtained may be converted into a sulfated oligohyaluronate in accordance with the present invention by treating it according to a per se known sulfation process. Generally, it is convenient to use a sulfuric acid-trimethylamine complex as the sulfating agent. The proportion of the aforesaid sulfating agent to the oligohyaluronic acid may be arbitrarily chosen according to the desired degree of sulfation. As to the reaction conditions, the reaction may be carried out in an aprotic solvent (e.g., dimethylformamide) at a temperature of 50 to 60°C for a period of time ranging from several tens of hours to several days.

The sulfated oligohyaluronate so formed can be purified according to a process which is per se known in the purification of oligosaccharides and, if necessary, involves gel filtration or fractional precipitation using acetone. The salt-forming reaction may be carried out either before or after the above-described purification process.

The sulfated oligohyaluronates obtained in the above-described manner exhibit certain moisture-retaining properties and skin permeability and, moreover, have the effect of inhibiting the local stratification of, for example, the epidermis. Furthermore, the characteristic feature which decisively distinguishes the sulfated oligohyaluronates from the sulfated products of chondroitins is that the sulfated oligohyaluronates can stay long in the skin at a high concentration through the medium of the hyaluronic acid receptors (CD₄₄) of cutaneous fibroblasts, and thereby exert their action. Accordingly, they can be used as active ingredients at least in the fields of cosmetics and dermatology.

More specifically, the sulfated oligohyaluronates of the present invention have excellent skin permeability and, moreover, can inhibit local stratification leading to the atrophy and keratinization of the epidermis. Furthermore, similarly to high-molecular weight hyaluronic acid sulfate, the sulfated oligohyaluronates of the present invention are essentially effective in preventing the infiltration of leukocytes into tissues and can hence be used for the prophylaxis or treatment of contact dermatitis and dark skin and for an atopic skin, a tender skin and the like.

The present invention is further illustrated by the following specific examples. However, these examples are not to limit the scope of the present invention.

### Example 1: Preparation of an oligohyaluronic acid from hyaluronic acid

7 g of high-molecular hyaluronic acid (with a molecular weight of 1,200 kDa) was dissolved in 350 ml of a 100 mM sodium acetate buffer 8pH 5.0), and hyaluronidase (derived from the bovine testicles; type IV; manufactured by Merck & Co., Inc.) was added thereto so as to give a final concentration of 150 U/ml. This mixture was incubated at 40°C for 3 days with the molecular weight monitored by viscometry. After the resulting reaction mixture was heated at 100°C for 20 minutes, the inactivated enzyme was removed by centrifugation. The supernatant was concentrated under reduced pressure to about 5 ml. 100 ml of ethanol was slowly added thereto and the precipitated oligohyaluronic acid was separated by centrifugation. The precipitate was purified by dissolving it in about 5 ml of a 1% sodium acetate solution, precipitating it with ethanol in the above-described manner, and repeating this procedure three times. The finally obtained precipitate was dried in vacuo to give 7 g of a white powder in a 100% yield.

Its molecular weight, which was calculated from its intrinsic viscosity measured with an Ubbelohde viscometer, was about 3,600 Da.

### Example 2: Preparation of an oligohyaluronic acid sulfate

4 g of a sulfuric acid trioxide-trimethylamine complex (manufactured by Aldrich Inc.) was dissolved in 30 ml of dimethylformamide. Then, 2 g of the oligohyaluronic acid obtained in Example 1 was reduced to a fine powder in a mortar, and suspended in the aforesaid dimethylformamide solution with stirring. Using a potting stirrer, the reaction was carried out at 60°C for 24 hours. With the progress of the reaction, the oligohyaluronic acid agglomerated to form a jelly-like mass. The supernatant was removed by centrifugation, and the precipitate was centrifugally washed twice with 10 ml portions of dimethylformamide. After the precipitate was dissolved in 10 ml of distilled water, the resulting solution was enclosed in a Spectra/Pore 6 (manufactured by Funakoshi Co., Ltd.; with a cut-off molecular weight of 1,000) and dialyzed overnight against 3 liters of distilled water. During this process, the outer liquid was replaced three times. The outside of the tube was covered with a powder of Sephadex G-200 to concentrate the inner liquid to about 5 ml. The inner liquid was transferred to a small beaker which could be sealed tightly, and trifluoroacetic acid was added thereto in an amount equal to 1.5 times the theoretical amount of hydroxyl groups. This mixture was stirred at room temperature for one hour to hydrolyze the trimethylamine complex. The resulting reaction mixture was enclosed again in a Spectra/Pore 6 and dialyzed against distilled water until the pH of the outer liquid became equal to that of distilled water. The inner liquid was transferred to a Spitz type centrifuge tube, sodium acetate was added so as to give a concentration of 1 wt.%, and 5 parts of acetone was added. Then, the precipitated hyaluronic acid sulfate was recovered by centrifugation (purification according to the acetone precipitation method). The above-described procedure was further repeated twice, and the resulting product was dried in vacuo to give about 1.2 g of a white powder of sulfated oligohyaluronate in an about 50% yield.

The sulfur content of the sulfated oligohyaluronate thus obtained was determined according to the flask combustion-dimethylsulfonazo III titration method, and the degree of sulfation was calculated from the measured value. Thus, it was found that about 20% of all hydroxyl groups present in the oligohyaluronic acid used as the starting material had been sulfated (and this corresponded to a molecular weight of about 4, 100 Da). Moreover, using an FT-IR spectrophotometer (Magna IR-550; manufactured by NICOLET Co., Ltd.), the aforesaid white powder was analyzed according to the KBr method, and the infrared absorption spectrum thus obtained is shown in FIG. 1.

### Examples 3 and 4: Preparation of oligohyaluronic acid sulfates having a high degree of sulfation

The procedure of Example 2 was repeated except that the ratio of the hyaluronic acid to the sulfuric acid trioxide-trimethylamine complex was changed from 1:2 to 1:5 and 1:10. Thus, oligohyaluronic acid sulfates having a degree of sulfation of 50% (corresponding to a molecular weight of about 5,000 Da; Example 3) and a degree of sulfation of 80-90% (corresponding to a molecular weight of about 6,000 Da; Example 4) were obtained in about 50% and about 60% yields, respectively.

### Example 5: Dialysis membrane permeability tests

Generally, skin permeability tends to decease rapidly with an increase in molecular weight. Accordingly, in order to obtain a measure of the skin permeability of a sulfated oligohyaluronate, each of the sulfated oligohyaluronates obtained in the above Examples 2, 3 and 4 was dialyzed through a routinely used dialysis membrane (i.e., a cellulose tube having a cut-off molecular weight of 10,000 Da). The sulfated oligohyaluronate present in the outer liquid was determined according to the cetyl pyridinium chloride (CPC) precipitation method.

The product of Example 2 (having a degree of sulfation of 20%) was detected in the outer liquid after the lapse of a dialysis time of only 5 minutes. Since the concentration in the outer liquid came to equilibrium with the concentration in the inner liquid after the lapse of one night, this product is believed to be freely permeable through the dialysis membrane.

The product of Example 3 (having a degree of sulfation of 50%) was scarcely detected in the outer liquid after the lapse of a dialysis time of 5 minutes. After the lapse of one night, about 1/3 of the sulfated oligohyaluronate present in the inner liquid was detected in the outer liquid. This indicates that the dialysis rate of the product of Example 3 having a higher degree of sulfation is suppressed as compared with the product of Example 2.

The product of Example 4 (having a degree of sulfation of 80-90%) became slightly turbid after being dialyzed overnight, indicating that this product is hardly permeable through the aforesaid dialysis membrane.

### Example 6: Evaluation of a local stratification-controlling effect by use of PAM212 cells

In the skin of human beings, the degeneration of the epidermis and the accumulation of stratum corneum increase markedly with aging, and this constitutes a cause for wrinkle formation. The only group of drugs having an anti-wrinkle effect are retinoids. Although they have a wide variety of effects, a keratinization-inhibiting effect is known as one of their effects on the epidermis. Since normal epidermal cells show an acceleration of keratinization dependent on calcium concentration, the degree of keratinization can be evaluated by using a change caused by a variation in calcium concentration as an index. Consequently, the PAM212 cell may be employed as an index for local stratification dependent on calcium ion That is, in the following experiment, retinol was used as a positive control for significantly inhibiting local stratification induced at a high calcium concentration, and its effect was compared with the effect of a hyaluronic acid derivative.

Moreover, an (unsulfated) oligohyaluronic acid was used for comparative purposes.

The PAM212 cell is a cell strain established from the keratinized cutaneous cells of a BALB/C mouse.

### (Testing procedure)

PAM212 cells were prepared at a density of 4 x 10⁴ cells per well and inoculated into 24 wells [Specifically, using a 2 ml pipet, one drop (50 µl) of a cell suspension containing 80 x 10⁴ cells per ml was added to each well. Moreover, using a 10 ml pipet, 0.5 ml of a culture medium was added to each well. Then, the four sides of the 24 wells were tapped so as to distribute the cells uniformly.] The culture medium was EMEM/10% untreated serum.

The cells were grown by standing culture for 2 days. After 80-90% confluency was reached, each test drug was added and its effect was compared with that of retinol. The drugs were previously prepared as 2(w/v)% aqueous solutions. Immediately before use, they were diluted tenfold with dimethyl sulfoxide (DMSO) to prepare 2 x 10⁻¹%, 2 x 10⁻²% and 2 x 10⁻³% DMSO solutions (drug-in-DMSO solutions). 5 µl each of these drug-in-DMSO solutions were added per milliliter of the culture medium to give final drug concentrations of 10⁻³%, 10⁻⁴% and 10⁻⁵%. Similarly, DMSO and retinol (at a final concentration of 10⁻⁷ M) were added. The culture medium used was EMEM/5% defatted serum.

The number of local stratification spots produced 24 hours after the addition of each drug was counted, and compared with the results obtained with DMSO and retinol. The number of local stratification spots obtained with DMSO was taken as 100%, and the degree of inhibition (%) by retinol was determined. On the basis of this degree of inhibition by retinol, each drug was rated as markedly effective, effective, indeterminable or ineffective when the degree of inhibition by the drug was 80-100%, 60-80%, 40-60% or less than 40%, respectively.

In order to confirm that the resulting effect was due to the test drug, measurements were made by replacing the number of cells by the amount of DNA for the purpose of examining the test drug for cytotoxicity. Since no cytotoxicity was observed, the drug which inhibited local stratification was judged to be an effective substance.

### (Test results)

When the local stratification-inhibiting effect of the oligohyaluronic acid was examined, this oligohyaluronic acid showed 81.1±14.1% at a concentration of 10⁻³% while the control group showed 100.1± 10.6%. This drug had no local stratification-inhibiting effect. (Retinol showed 9.5±2.1% at a concentration of 10⁻⁷%.)

On the other hand, when the local stratification-inhibiting effect of the sulfated oligohyaluronate (obtained in Example 2) was examined, this sulfated oligohyaluronate showed 86.5±23.1% at a concentration of 10⁻⁴% while the control group showed 99.9±14.7%. Since a significant difference (unpaired t-test) was noted as compared with the control group, it was confirmed that this drug had a local stratification-inhibiting effect. (Retinol showed 9.5±2.1% at a concentration of 10⁻⁷%.)

As can be seen from the above-described results, the sulfated oligohyaluronates of the present invention which have certain skin permeability and can inhibit local stratification are useful, for example, as active ingredients for cosmetics.

## Claims

1. A sulfated oligohyaluronate composed of repeating units represented by the following formula (I) or a salt thereof. 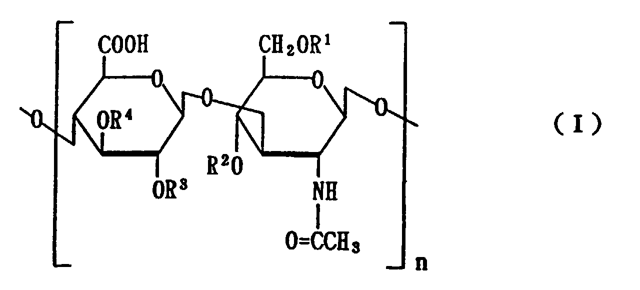 wherein
R¹, R², R³ and R⁴ each independently represent a hydrogen atom or an SO₃H group;
n represents an average value corresponding to a viscosity-average molecular weight of 1,000 to 50,000; and
10 to 90% of the total number of the radicals represented by R¹, R², R³ and R⁴ are occupied by SO₃H groups.

2. A sulfated oligohyaluronate or a salt thereof as claimed in claim 1 wherein n in formula (I) represents an average value corresponding to a viscosity-average molecular weight of 1,000 to 10,000.

3. A sulfated oligohyaluronate or a salt thereof as claimed in claim 1 or 2 wherein 20 to 50% of the total number of R¹, R², R³ and R⁴ are occupied by SO₃H groups.
